# EUROPEAN PATENT SPECIFICATION

(11) **EP 3 653 183 B1**
(45) Date of publication and mention of the grant of the patent: **15.09.2021**
(21) Application number: 19209032.2
(22) Date of filing: 14.11.2019
(51) Int. Cl.: A61F 9/00, G02C 7/04

(54) **OCULAR INSERT**
AUGENEINSATZ
DISPOSITIF D'INSERTION OCULAIRE

(30) Priority: 14.11.2018 US 201862760929 P
(43) Date of publication of application: 20.05.2020
(73) Proprietor: Industrial Technology Research Institute, Hsinchu 31040 (TW)
(72) Inventor: TENG, Yun-Chung, 846 Kaohsiung City (TW); LIOU, Yu-Bing, 300 Hsinchu City (TW); HSU, Hsin-Yi, 320 Taoyuan City (TW); SHEN, Ying-Wen, 350 Miaoli County (TW); CHEN, Sen-Lu, 350 Miaoli County (TW); WANG, Yu-Chi, 221 New Taipei City (TW); SHEN, Hsin-Hsin, 116 Taipei City (TW)
(74) Representative: Becker & Kurig Partnerschaft Patentanwälte PartmbB

(56) References cited:
- EP-A1- 2 397 114
- WO-A1-2016/031093
- CA-A1- 2 764 063
- CN-C- 100 510 846
- US-A1- 2017 281 408

## Description

### TECHNICAL FIELD

The disclosure relates to an eye wearing device, in particular to an eye wearing device for drug delivery and a use method thereof.

### BACKGROUND

The existing methods for improving bioavailability of eyedrops mainly include drug/dosage form development and implantation/wearing drug delivery. However, for a slow release composite medical material, specific categories of drugs are generally made into nanoparticles, hydrogels or polymer carriers, and needs to be bound with a specific drug, and therefore the storage and the manufacturing processes are restricted to the drug, and it is not applicable for personalized drug. Additionally, the problem of excessively fast release often occurs in known technologies. For storage volume or bioavailability improvement, a good fixing and oxygen permeating design is needed for the connection with the eye surface. Meanwhile, there are many limitations in drug selection and matching in the known art, and the range of currently available excipients, pH value and osmotic pressure is still very narrow even for eyedrops. Clinically, the existing medical material for drug delivery also faces the problems of low bioavailability and poor drug compliance. Therefore, the development of an eye wearing device capable of improving the drug release condition, realizing simple and convenient operation and flexibly matched with personalized drugs is in urgent need.

A device according to the preamble of claim 1 is known from the document CA2764063.

### SUMMARY

The disclosure provides an eye wearing device capable of being used for storing, slowly releasing and supplementing a treatment drug without hindering the vision of a user.

The eye wearing device of the disclosure includes a body, at least one reservoir and at least one microchannel. The body includes a first surface, a second surface, a center, a first outer edge of the first surface and a second outer edge of the second surface. The at least one reservoir is arranged in the body and is configured to load a drug. The at least one microchannel is arranged in a position close to the first outer edge of the first surface and the second outer edge of the second surface. One end of the microchannel is connected to the reservoir so as to fill the drug into the reservoir. The other end of the microchannel is an opening facing the edge of the eye wearing device and is configured to be in contact with a cornea and/or a sclera. The opening is connected to the first outer edge of the first surface and the second outer edge of the second surface. The average diameter of the cross section of the microchannel is smaller than or equal to the average diameter of the cross section of the reservoir. The average curvature radius of the eye wearing device is 6 mm to 15 mm. The eye wearing device is worn on the cornea and/or the sclera of the user through the second surface.

Based on the above, the eye wearing device of the disclosure includes at least one reservoir and at least one microchannel, in which the microchannel can eliminate stoppers such as bubbles and promote drug filling or supplementation, and can further be matched with material properties so as to enhance the drug supplementing capability through blinking. The microchannel is subjected to specific surface treatment so as to enhance the efficiency. Therefore, the eye wearing device of the disclosure can be used for effectively treating eye diseases in an auxiliary way, is particularly favourable for the use of personalized drugs, and can be used for storing, slowly releasing and supplementing the treatment drug without hindering the vision of the user.

To make the features and advantages of the disclosure clear and easy to understand, the following gives a detailed description of embodiments with reference to accompanying drawings.

### BRIEF DESCRIPTION OF THE DRAWINGS

FIG. 1A is a top view schematic diagram of an eye wearing device according to the first embodiment of the disclosure. FIG. 1B is a straight cutting cross-section schematic diagram along a tangent line A-A' in FIG. 1A. FIG. 1C is a straight cutting cross-section schematic diagram along a tangent line B-B' in FIG. 1A.
FIG. 2 is a top view schematic diagram of an eye wearing device according to the second embodiment of the disclosure.
FIG. 3A to FIG. 3B are stereoscopic schematic diagrams of an eye wearing device according to the disclosure.
FIG. 4A to FIG. 4C are stereoscopic schematic diagrams of another eye wearing device according to the disclosure.
FIG. 5A to FIG. 5B are schematic diagrams of a configuration mode of a reservoir and a microchannel in an eye wearing device according to the third embodiment of the disclosure, in which FIG. 5A is a top view schematic diagram, and FIG. 5B is a straight cutting cross-section schematic diagram along a tangent line C-C' in FIG. 5A.
FIG. 6A to FIG. 6C are schematic diagrams of a configuration mode of a reservoir and a microchannel in an eye wearing device according to the fourth embodiment of the disclosure, in which FIG. 6A is a top view schematic diagram, and FIG. 6B and FIG. 6C are straight cutting cross-section schematic diagrams along a tangent line D-D' in FIG. 6A. FIG. 6B and FIG. 6C respectively represent different modes of a gradually reducing microchannel. FIG. 6B is a smooth gradually reducing mode. FIG. 6C is a stepped gradually reducing mode.
FIG. 7A to FIG. 7D are schematic diagrams of a package design of an eye wearing device according to the disclosure, in which FIG. 7A is a cross-section schematic diagram, FIG. 7B is a top view schematic diagram, FIG. 7C is a stereoscopic schematic diagram, and FIG. 7D is a local amplification schematic diagram.
FIG. 8A, FIG. 8B and FIG. 8C are schematic diagrams of another package design of the eye wearing device according to the disclosure.
FIG. 9A and FIG. 9B are schematic diagrams of yet another package design of the eye wearing device according to the disclosure, in which FIG. 9A is a stereoscopic schematic diagram, and FIG. 9B is a cross-section schematic diagram.

### DETAILED DESCRIPTION OF DISCLOSED EMBODIMENTS

The following embodiments are described in details with reference to accompanying drawings, but the provided embodiments are not intended to limit the scope covered by the present disclosure. In addition, the drawings are drawn only for the purpose of description, and are not drawn according to original sizes. For ease of understanding, same elements in the following description are described by using the same signs. Terms such as "includes", "comprises", and "having" used herein are all inclusive terms, namely, mean "includes but not limited to". In addition, the directional terms mentioned herein, like "above" and "below", are only used to refer to the directions in the accompanying drawings and are not intended to limit the disclosure. In addition, the quantities and shapes mentioned in the specification are only used to specifically describe the disclosure to facilitate understanding of contents of the disclosure, and are not intended to limit the disclosure.

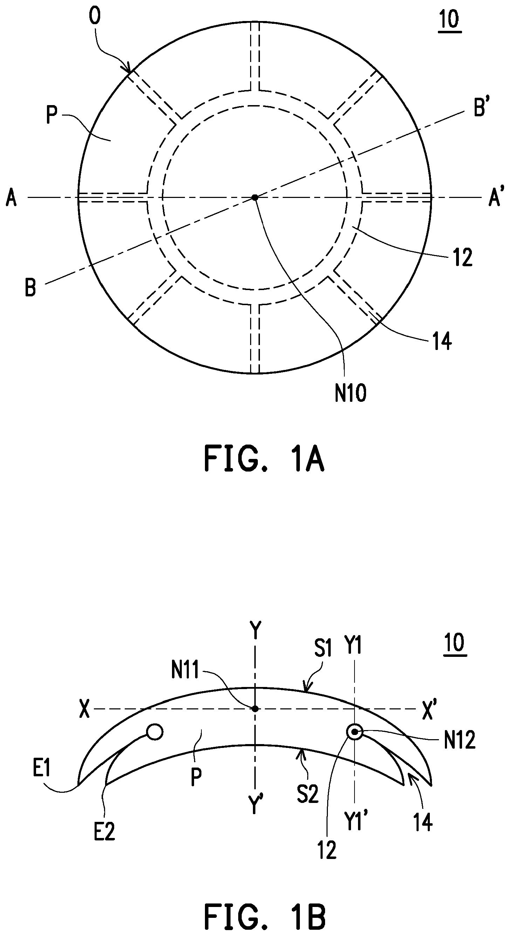

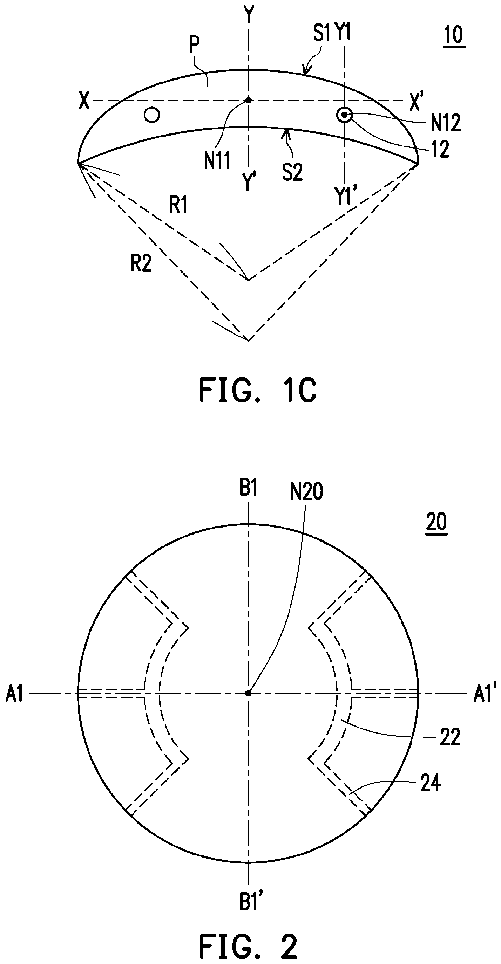

FIG. 1A is a top view schematic diagram of an eye wearing device according to the first embodiment of the disclosure. FIG. 1B is a straight cutting cross-section schematic diagram along a tangent line A-A' in FIG. 1A. FIG. 1C is a straight cutting cross-section schematic diagram along a tangent line B-B' in FIG. 1A.

Referring to FIG. 1A, FIG. 1B and FIG. 1C altogether, an eye wearing device 10 can include a body P, at least one reservoir 12 and at least one microchannel 14. The body P can structurally include a first surface S1, a second surface S2, a center N10 and N11, an outer edge E1 of the first surface S1 and an outer edge E2 of the second surface S2. The above-mentioned center N10 refers to a center point of the body P defined by the top view schematic diagram of FIG. 1A (can be defined by an intersection point of the tangent line A-A' and the tangent line B-B' in FIG. 1A), and the center N11 refers to a center point of the body P defined by the cross-section schematic diagrams of FIG. 1B and FIG. 1C. Referring to FIG. 1B and FIG. 1C, Y-Y' is a longitudinal axis line penetrating through the body P through the center N11 in a longitudinal direction, X-X' is a horizontal axis line penetrating through the body P through the center N11 in a horizontal direction, and the longitudinal axis line Y-Y' and the horizontal axis line X-X' are vertical to each other.

The average curvature radius of the eye wearing device 10 can be about 6 mm to 15 mm, and for example, can be 6 mm to 14.5 mm, 6.5 mm to 13 mm, 6.5 mm to 12 mm, 7 mm to 9 mm, 8.5 mm to 10.5 mm, 7 mm to 10 mm, etc., but the disclosure is not limited thereto. Further, referring to FIG. 1C, the first surface S1 and the second surface S2 of the eye wearing device 10 can respectively have different average curvature radii R1 and R2, and the first average curvature radius R1 of the first surface S1 is smaller than the second average curvature radius R2 of the second surface S2. For example, the first average curvature radius R1 can be about 6.0 mm to 14.8 mm, for example, 6 mm to 14.5 mm, 6 mm to 13 mm, 6.5 mm to 12 mm, 7 mm to 11.5 mm, 7.5 mm to 11 mm, 8 mm to 10.5 mm, 7 mm to 10 mm, etc., but the disclosure is not limited thereto. The second average curvature radius R2 can be about 6.2 mm to 15.0 mm, for example, 6.5 mm to 14.5 mm, 6.5 mm to 13 mm, 7 mm to 12.5 mm, 7 mm to 12 mm, 7.5 mm to 11.5 mm, 8 mm to 11 mm, 8 mm to 10 mm, etc., but the disclosure is not limited thereto. Additionally, the eye wearing device 10 uses the second surface S2 to be in contact with the cornea and/or the sclera of the user when being worn.

Additionally, a material of the eye wearing device 10 can include bio-derived polymers, non-bio-derived polymers or a combination thereof, in which the bio-derived polymers can include collagen, gelatin, chitin, cellulose or a combination thereof, but the disclosure is not limited thereto. The non-bio-derived polymers can include polyethylene glycol (PEG), propylene glycol diacrylate (PPGDA), polydimethylsiloxane (PDMS), poly(methyl methacrylate) (PMMA), poly(hydroxyethyl methacrylate) (PHEMA) or a combination thereof, but the disclosure is not limited thereto.

The reservoir 12 is arranged in the body P, and can be configured to load the drug. The microchannel 14 is arranged in the position close to the outer edge E1 of the first surface S1 and the outer edge E2 of the second surface S2, and can be configured to fill the drug into the reservoir 12. The reservoir 12 and the microchannel 14 can further be subjected to hydrophilic or anti-sticking modification treatment on the surface, so that drug filling or bubble elimination is promoted through capillary action, and material properties (such as softness and elasticity) can be further matched so as to enhance the drug supplementing capability through blinking. The hydrophilic modification treatment mode can include the steps that hydrophilic polymers, ionic functional groups or interface active agents are mixed into a substrate, are immersed/coated onto the surface of the substrate or are grafted onto the surface of the substrate through chemical reaction, the surface of a substance can also be subjected to oxidization, crosslinking, easy-to-react functional group addition or micro structure change treatment by using plasma, ultraviolet light, heat treatment or other reactant gas, but the disclosure is not limited thereto. In detail, the hydrophilic polymers can include polyacrylamide (PAM or PAAM), polyethylene glycol (PEG), etc., the ionic functional groups can include primary/secondary amine, carboxylate radicals, etc., and the interface active agents can include sodium dodecyl sulfate (SDS), polyethylene glycol p-(1,1,3,3-tetramethylbutyl)-phenyl ether (Triton X-100), {[3-(Dodecanoylamino)propyl](dimethyl)ammonio}acetate, etc., but the disclosure is not limited thereto. The mode of the anti-sticking modification treatment is similar to that of the hydrophilic modification, and is not repeated herein, but the disclosure is not limited thereto.

In the present embodiment, the reservoir 12 can be in a ring shape, an arc shape, a line shape or a combination thereof, but the disclosure is not limited thereto. The reservoir 12 is designed to be in a position not hindering the vision in the eye wearing device 10. Referring to FIG. 1B and FIG. 1C, Y1-Y1' is a longitudinal axis line penetrating through the body P through the center N12 of the reservoir 12 in a longitudinal direction. For example, the center N12 of the reservoir 12 can be arranged in a position 4 mm to 14 mm away from the center N11 of the body, the center N12 of the reservoir 12 can also be arranged in a position about 4.5 mm to 14 mm, 6 mm to 14 mm, 4 mm to 12 mm, 8 mm to 12 mm, 10 mm to 12 mm, 6.5 mm to 10 mm, 8 mm to 10 mm, 7 mm to 11 mm, 5.5 mm to 10.5 mm, 4.5 mm to 8 mm away from the center N11 of the body, but the disclosure is not limited thereto. In other words, the shape of the reservoir 12 and the arrangement position of the reservoir 12 in the eye wearing device 10 can be adjusted according to practical requirements.

In the present embodiment, one end of the microchannel 14 can be connected to the reservoir 12, the other end faces an opening O arranged at the edge of the eye wearing device 10, and is configured to be in contact with the cornea and/or the sclera, and the opening O is connected to the outer edge E1 of the first surface S1 and the outer edge E2 of the second surface S2. The configuration mode of the microchannel 14 can be adjusted according to practical requirements. For example, the configuration mode of the microchannel 14 in the eye wearing device 10 can include but is not limited to a radial type. Although FIG. 1A shows eight microchannels 14, the configuration number of the microchannel 14 of the disclosure is not limited thereto, and can be adjusted to be 16, 14, 12, 10 or 6, etc. according to practical requirements.

Additionally, a lubricating material, such as mucoprotein, polyethylenimine, polyethylene glycol, polyacrylic acid, polymethacrylic acid, polyitaconic acid, polymaleic acid, carboxymethyl cellulose (CMC), hydroxypropyl methylcellulose (HPMC), polyvinylpyrrolidone, polyacrylamide, poleyvinylalcohol, hyaluronic acid, dextran, poly 2-hydroxyethyl methacrylate (poly HEMA), poly sulfonates, polylactate, urea, phosphoryl choline or a combination thereof can be further coated on the surface of the eye wearing device 10. In addition, the lubricating material can also be hydrophilic polypeptides, for example, 75 or above weight percent of amino acids of the polypeptides are selected from the group consisting of aspartic acid (Asp or D), glutamic acid (Glu or E), histidine (His or H), lysine (Lys or K), asparagine (Asn or N), glutamine (Gln or Q), arginine (Arg or R), serine (Ser or S), threonine (Thr or T) and tyrosine (Tyr or Y), but the disclosure is not limited thereto. The material is mainly directed to improve wearing comfort, and can avoid moisture evaporation.

Referring to FIG. 1A, FIG. 1B and FIG. 1C altogether, the width of the reservoir 12 can be 30 µm to 5 mm, for example, 30 µm to 50 µm, 50 µm to 70 µm, 70 µm to 100 µm, 100 µm to 4.5 mm, 500 µm to 4.5 mm, 1 mm to 4 mm, 1.5 mm to 4 mm, 1.5 mm to 3.5 mm, 2 mm to 3.5 mm, 2.5 mm to 3.5 mm, 2.5 mm to 3 mm, etc., but the disclosure is not limited thereto. The height of the reservoir 12 can be 10 µm to 200 µm, for example, 20 µm to 180 µm, 30 µm to 150 µm, 50 µm to 120 µm, 50 µm to 100 µm, 60 µm to 80 µm, etc., but the disclosure is not limited thereto. The total volume of the drug loaded in the reservoir 12 can be 0.002 µL to 20 µL, for example, 0.05 µL to 20 µL, 0.1 µL to 20 µL, 0.5 µL to 20 µL, 1 µL to 20 µL, 2 µL to 20 µL, 5 µL to 20 µL, 10 µL to 20 µL, 5 µL to 15 µL, 5 µl to 10 µL, etc., but the disclosure is not limited thereto.

Additionally, the average diameter of the cross section of the microchannel 14 can be 20 µm to 150 µm, for example, 30 µm to 150 µm, 50 µm to 150 µm, 50 µm to 120 µm, 50 µm to 100 µm, 50 µm to 80 µm, 80 µm to 120 µm, 100 µm to 150 µm, etc., but the disclosure is not limited thereto. The average diameter of the cross section of the microchannel 14 can be smaller than or equal to the average diameter of the cross section of the reservoir 12, in which the diameter of one end of the microchannel 14 connected to the reservoir 12 can be 10 µm to 200 µm, for example, 10 µm to 20 µm, 20 µm to 50 µm, 50 µm to 100 µm, 100 µm to 150 µm, 80 µm to 200 µm, etc., but the disclosure is not limited thereto. The diameter of one end of the microchannel in contact with the cornea and/or the sclera can be 40 µm to 200 µm, for example, 50 µm to 180 µm, 60 µm to 160 µm, 70 µm to 150 µm, 80 µm to 140 µm, 90 µm to 130 µm, 100 µm to 120 µm, etc., but the disclosure is not limited thereto.

Referring to FIG. 1A, FIG. 1B and FIG. 1C altogether, the appearance of the eye wearing device 10 can include but is not limited to a round shape. The diameter can be about 12 mm to 20 mm, for example, 12 mm to 15 mm, 15 mm to 18 mm, 18 mm to 20 mm, 12 mm to 18 mm, 15 mm to 20 mm, 13 mm to 15 mm, etc., but the disclosure is not limited thereto. The average thickness of the eye wearing device can be about 20 µm to 400 µm, for example, 30 µm to 350 µm, 50 µm to 300 µm, 50 µm to 250 µm, 80 µm to 320 µm, 100 µm to 300 µm, 150 µm to 300 µm, 150 µm to 200 µm, etc., but the disclosure is not limited thereto.

FIG. 2 is a top view schematic diagram of an eye wearing device according to the second embodiment of the disclosure. The second embodiment in FIG. 2 is similar to the first embodiment in FIG. 1A, so that the specifications and configuration of identical assemblies are not repeated herein.

In the second embodiment in the FIG. 2, a center N20 refers to a center point of a body defined by the top view schematic diagram of FIG. 2 (can be defined by an intersection point of a tangent line A-A' and a tangent line B-B' in FIG. 2). The difference between FIG. 2 and the first embodiment is that a reservoir 22 in the second embodiment is in an arc shape, for example, in a circular arc shape, and is configured by using the center N20 of an eye wearing device 20 as the center. In other words, in the first embodiment in FIG. 1A, the eye wearing device includes a ring-shaped reservoir 12, and in the second embodiment in FIG. 2, the eye wearing device includes two arc-shaped reservoirs 22, but the disclosure is not limited thereto. The design is directed to not hindering the vision. The shapes, the configuration positions and the configuration number of the reservoirs 22 can be adjusted according to practical requirements. As shown in FIG. 2, the reservoirs 22 can be symmetrically configured by using the center N20 of the eye wearing device 20 as the symmetrical center, but the disclosure is not limited thereto. The reservoirs 22 can also be in an unsymmetrical configuration mode.

As shown in FIG. 2, one end of a microchannel 24 is connected to the reservoir 22, the other end is an opening facing the edge of the eye wearing device, and can include but is not limited to radial configuration. In addition, FIG. 2 shows six microchannels 24, and each reservoir 22 is respectively connected to three microchannels 24, but the disclosure is not limited thereto, and the configuration number of the microchannels 24 can be adjusted according to practical requirements. For example, each reservoir 22 can also be connected to two, four, five, six and other number of microchannels 24, but the disclosure is not limited thereto. In addition, as mentioned above, the reservoir 22 can also be in an unsymmetrical configuration mode. For example, one reservoir 22 can be matched with two microchannels 24, and the other reservoir 22 can be matched with three microchannels 24. In other words, the numbers of the microchannels 24 matched with the two reservoirs 22 can also be different.

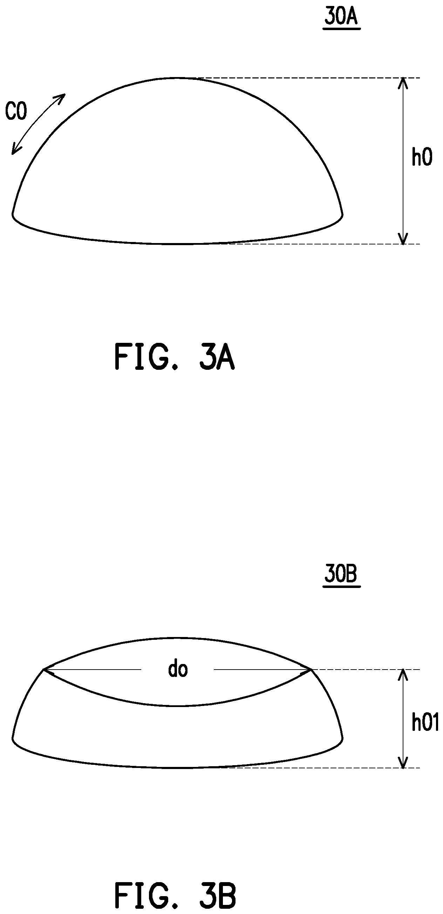

FIG. 3A to FIG. 3B are stereoscopic schematic diagrams of the eye wearing device according to the disclosure.

Referring to FIG. 3A, an eye wearing device 30A can be in a full lens type. The average curvature radius of a curve surface C0 can be about 6 mm to 15 mm, for example, 6 mm to 12 mm, 6 mm to 10 mm, 7 mm to 14 mm, 7 mm to 12 mm, 8 mm to 12 mm, 8 mm to 10 mm, etc., but the disclosure is not limited thereto. Additionally, the integral height from the top to the bottom of the full lens type eye wearing device 30A can be about 0.3 mm to 6.2 mm, for example, 0.5 mm to 3 mm, 1 mm to 4.5 mm, 2 mm to 5 mm, 2 mm to 4.5 mm, 2.5 mm to 5 mm, 4 mm to 6 mm, etc., but the disclosure is not limited thereto. Referring to FIG. 3B, an eye wearing device 30B can also be in an outer ring shape, in which the diameter d0 of a ring-shaped opening can be about 6 mm to 13 mm, for example, 6 mm to 12 mm, 7 mm to 12 mm, 8 mm to 12 mm, 8 mm to 10 mm, etc., but the disclosure is not limited thereto. Additionally, the height h01 of the ring shape can be about 0.2 mm to 6 mm, for example, 0.2 mm to 2 mm, 0.5 mm to 5 mm, 1 mm to 4.5 mm, 1.5 mm to 4 mm, 2 mm to 3.5 mm, 2.5 mm to 3 mm, 3 mm to 5 mm, etc., but the disclosure is not limited thereto. In detail, if the full lens type eye wearing device 30A in FIG. 3A is cut at a position of height h01 of the FIG. 3B, the outer ring-shaped eye wearing device 30B in FIG. 3B can be formed.

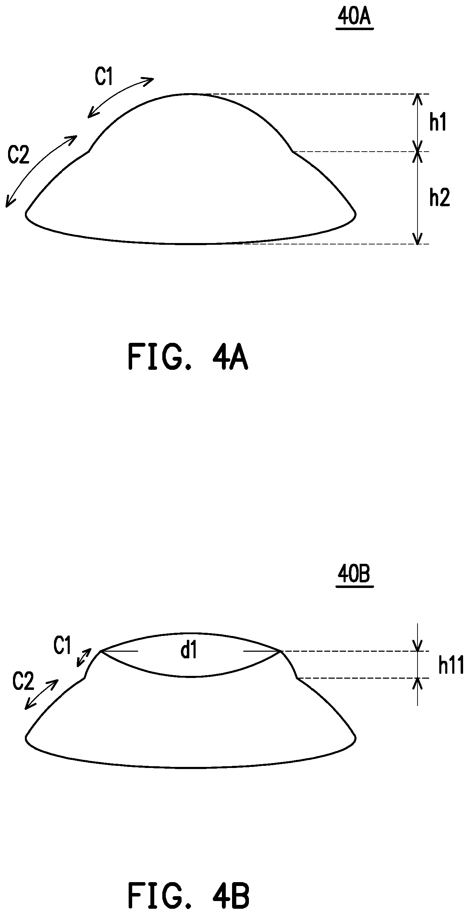

FIG. 4A to FIG. 4C are stereoscopic schematic diagrams of another eye wearing device according to the disclosure.

Referring to FIG. 4A to FIG. 4C, eye wearing devices 40A, 40B and 40C can be of a sclera-contact wearing type. Referring to FIG. 4A, the eye wearing device 40A can be of a sclera-contact wearing full lens type, and has two layers of curve surfaces C1 and C2. The curvature radius of the curve surface C1 can be about 6 mm to 9 mm, for example, 6.5 mm to 8.5 mm, 7 mm to 8.5 mm, 7.5 mm to 9 mm, etc., but the disclosure is not limited thereto. The curvature radius of the curve surface C2 can be about 7 mm to 15 mm, for example, 8 mm to 14 mm, 8 mm to 12 mm, 10 mm to 12 mm, etc., but the disclosure is not limited thereto. The height h1 of the lens with the curve surface C1 can be about 0.3 mm to 6.2 mm, for example, 0.5 to 2 mm, 1 mm to 6 mm, 2 mm to 5 mm, 3 mm to 4 mm, etc., but the disclosure is not limited thereto. The height h2 of the lens with the curve surface C2 can be about 0.2 mm to 10 mm, for example, 0.2 mm to 3 mm, 1 mm to 9 mm, 2 mm to 8 mm, 3 mm to 7 mm, 4 mm to 6 mm, etc., but the disclosure is not limited thereto.

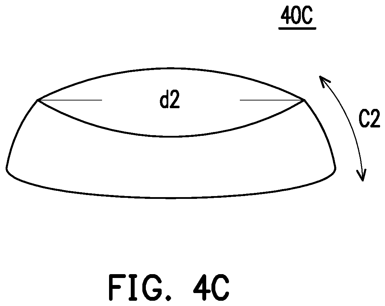

Referring further to FIG. 4B and FIG. 4C, the eye wearing devices 40B and 40C can also be of an outer ring form of the sclera-contact wearing type. In detail, if the full lens type eye wearing device 40A in FIG. 4A is cut at a position of height h11 of the outer ring with the curve surface C1 in FIG. 4B, the outer ring type eye wearing device 40B in FIG. 4B can be formed. If the full lens type eye wearing device 40A in FIG. 4A is cut in a juncture position of a part with the curve surface C1 and a part with the curve surface C2 to cut off the part with the curve surface C1, and the outer ring type eye wearing device 40C in FIG. 4C can be formed. As shown in FIG. 4B, the diameter d1 in a ring-shaped opening can be about 6 mm to 13 mm, for example, 6 mm to 12 mm, 7 mm to 12 mm, 8 mm to 12 mm, 8 mm to 10 mm, etc., but the disclosure is not limited thereto. Additionally, the height h11 of the outer ring of the part with the curve surface C1 can be about 0.2 mm to 5 mm, for example, 0.5 mm to 4.5 mm, 1 mm to 4 mm, 1.5 mm to 3.5 mm, 2 mm to 3.5 mm, 2.5 mm to 3 mm, 3 mm to 5 mm, etc., but the disclosure is not limited thereto. As shown in FIG. 4C, the diameter d2 of the ring-shaped opening can be about 9 mm to 19 mm, for example, 9 mm to 11 mm, 10 mm to 18 mm, 10.5 mm to 17.5 mm, 11 mm to 17 mm, 12 mm to 16 mm, 12.5 mm to 15.5 mm, 13 mm to 15 mm, etc., but the disclosure is not limited thereto.

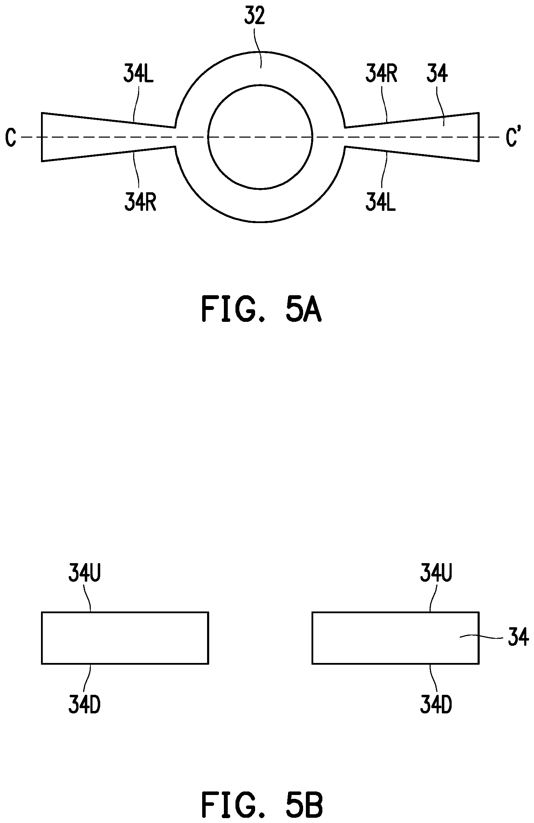

FIG. 5A to FIG. 5B are schematic diagrams of a configuration mode of a reservoir and a microchannel in an eye wearing device according to the third embodiment of the disclosure, in which FIG. 5A is a top view schematic diagram, and FIG. 5B is a straight cutting cross-section schematic diagram along a tangent line C-C' in FIG. 5A. The third embodiment in FIG. 5A and FIG. 5B is similar to the first embodiment of FIG. 1A, so that the specifications and the configuration of identical assemblies are not repeated herein. It should be noted that, in order to clearly show the mode configuration of a reservoir 32 and microchannels 34, the drawing of an edge contour line of the eye wearing device is omitted in FIG. 5A and FIG. 5B, and only the reservoir 32 and the microchannels 34 are drawn. Additionally, only two microchannels 34 are drawn in this part to be connected to the reservoir 32, but the number of the microchannels 34 can still be adjusted according to practical requirements, and the disclosure is not limited thereto. For example, two, four, six, eight, ten and other number of microchannels 34 can be arranged to be connected to the reservoir 32.

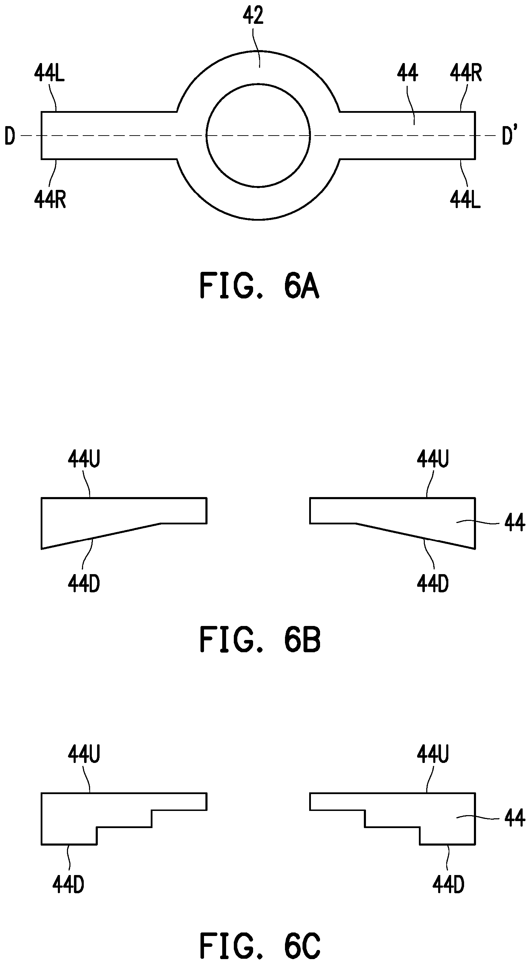

Referring to FIG. 5A and FIG. 5B, the diameter of the microchannel 34 is gradually reduced from the edge of the eye wearing device to the reservoir 32 so as to enhance a drug filling and bubble eliminating mechanism promoted through capillary action. In the present embodiment, the diameter of the microchannel 34 can be gradually reduced from the edge of the eye wearing device in the top view direction of the eye wearing device to the reservoir 32. Therefore, in the top view schematic diagram of FIG. 5A, the diameter of the microchannel 34 is gradually reduced from the edge of the eye wearing device to the reservoir 32. In the cross-section schematic diagram of FIG. 5B, the diameters of the microchannels 34 are uniform. More specifically, through the top view schematic diagram of FIG. 5A, if an angle from one end, near the edge of the eye wearing device, of the microchannel 34 to the reservoir 32 is used as an inspection view angle, two side walls of the microchannels 34 are respectively defined as a left side wall 34L and a right side wall 34R. As shown in FIG. 5A, the left side wall 34L and the right side wall 34R of the microchannel 34 are in a mutually and gradually approaching mode. In the cross-section schematic diagram of FIG. 5B, the distance between an upper side wall 34U and a lower side wall 34D of the microchannel 34 keeps unchanged. In other words, in the present embodiment, the width between the left side wall 34L and the right side wall 34R of the microchannel 34 is gradually reduced from the edge of the eye wearing device to the reservoir 32, but the height between the upper side wall 34U and the lower side wall 34D of the microchannel 34 keeps unchanged from the edge of the eye wearing device to the reservoir 32. The disclosure is not limited thereto. For example, the diameter of the microchannel 34 can also be gradually reduced from the edge of the eye wearing device to the reservoir 32 in the cross-sectional direction of the eye wearing device, and the mode will be illustrated in detail thereafter by referring to FIG. 6A, FIG. 6B and FIG. 6C.

FIG. 6A to FIG. 6C are schematic diagrams of a configuration mode of a reservoir and a microchannel in an eye wearing device according to the fourth embodiment of the disclosure, in which FIG. 6A is a top view schematic diagram, and FIG. 6B and FIG. 6C are straight cutting cross-section schematic diagrams along a tangent line D-D' in FIG. 6A. Only FIG. 6B and FIG. 6C respectively represent different modes of a gradually reducing microchannel. FIG. 6B is a cross-section schematic diagram of a smooth gradually reducing mode. FIG. 6C is a cross-section schematic diagram of a stepped gradually reducing mode. The fourth embodiment in FIG. 6A, FIG. 6B and FIG. 6C is similar to the first embodiment in FIG. 1A, so that the specifications and the configuration of the identical assemblies are not repeated herein. It should be noted that, in order to clearly show the mode configuration of a reservoir 42 and microchannels 44, the drawing of an edge contour line of the eye wearing device is omitted in FIG. 6A, FIG. 6B and FIG. 6C, and only the reservoir 42 and the microchannels 44 are drawn. Additionally, only two microchannels 44 are drawn in this part to be connected to the reservoir 42, but the number of the microchannels 44 can still be adjusted according to practical requirements, and the disclosure is not limited thereto. For example, two, four, six, eight, ten and other number of microchannels 44 can be arranged to be connected to the reservoir 42.

Referring to FIG. 6A, FIG. 6B and FIG. 6C at the same time, the diameter of the microchannel 44 is gradually reduced from the edge of the eye wearing device to the reservoir 42 so as to enhance a drug filling and bubble eliminating mechanism promoted through capillary action. In the present embodiment, the diameter of the microchannel 44 can be gradually reduced from the edge of the eye wearing device in the cross-section direction of the eye wearing device to the reservoir 42. Therefore, in the top view schematic diagram of FIG. 6A, the diameters of the microchannel 44s are uniform. In the cross-section schematic diagrams of FIG. 6B and FIG. 6C, the diameter of the microchannel 44 has a gradually reducing trend from the edge of the eye wearing device to the reservoir 42. More specifically, through the top view schematic diagram of FIG. 6A, if an angle from one end, near the edge of the eye wearing device, of the microchannel 44 to the reservoir 42 is used as an inspection view angle, two side walls of the microchannels 44 are respectively defined as a left side wall 44L and a right side wall 44R. As shown in FIG. 6A, the distance between the left side wall 44L and the right side wall 44R of the microchannel 44 keeps unchanged. In the cross-section schematic diagrams of FIG. 6B and FIG. 6C, a lower side wall 44D of the microchannel 44 shows a trend of gradually approaching an upper side wall 44U. In other words, in the present embodiment, the width between the left side wall 44L and the right side wall 44R of the microchannel 44 keeps unchanged from the edge of the eye wearing device to the reservoir 42. The height between the upper side wall 44U and the lower side wall 44D of the microchannel 44 is gradually reduced from the edge of the eye wearing device to the reservoir 42. It should be noted that in the present embodiment, the way that the diameter of the microchannel 44 is gradually reduced from the edge of the eye wearing device to the reservoir 42 in a smooth and gradually reducing manner (as shown in FIG. 6B) or a step-like and gradually reducing manner (as shown in FIG. 6C), but the present disclosure provides no limitation to the gradually reducing manner.

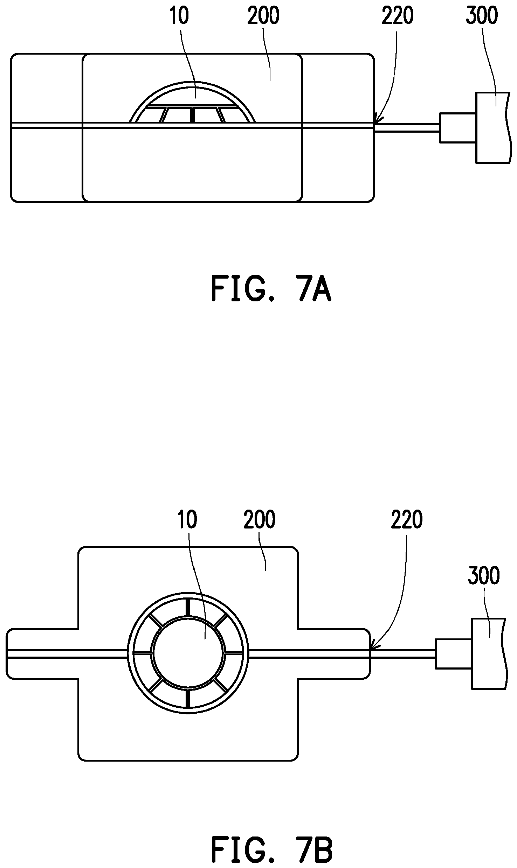

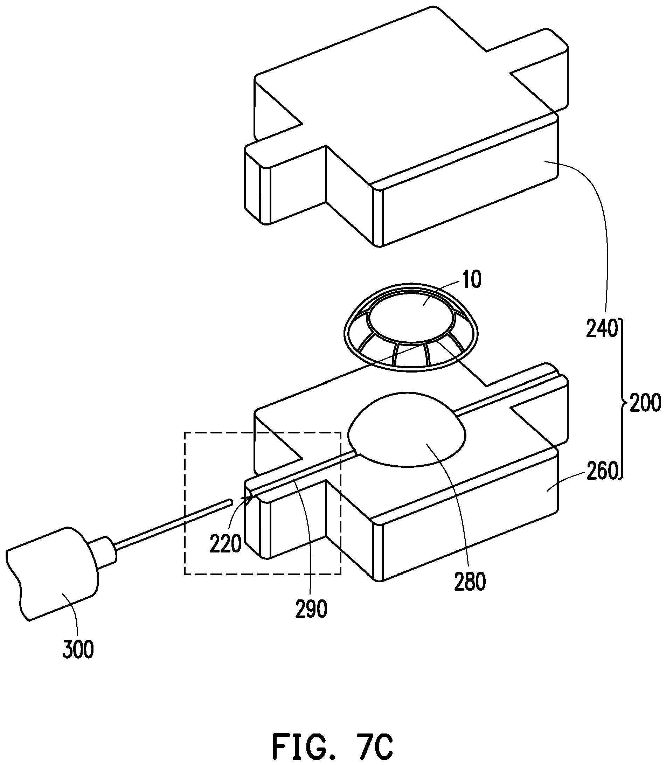

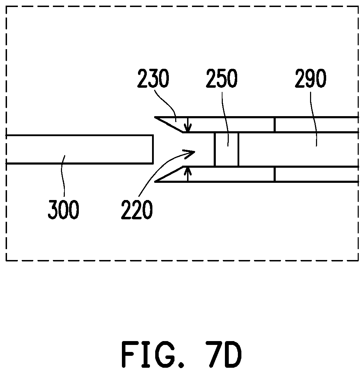

FIG. 7A to FIG. 7D are schematic diagrams of a package design of the eye wearing device according to the disclosure, in which FIG. 7A is a cross-section schematic diagram, FIG. 7B is a top view schematic diagram, FIG. 7C is a stereoscopic schematic diagram, and FIG. 7D is a local amplification schematic diagram.

Referring to FIG. 7A, FIG. 7B and FIG. 7C altogether, the eye wearing device 10 can be packaged in a hard packaging clamp 200 so that the eye wearing device 10 can be supported and is prevented from collapsing and deforming, the completeness of the microchannel can be maintained, and the drug filling precision can be improved. The eye wearing device 10 is used as an example in FIG. 7A, FIG. 7B and FIG. 7C, but the disclosure is not limited thereto. For example, eye wearing devices 20, 30A, 30B, 40A, 40B or 40C and the like can also be used. The packaging mode can use vacuum and negative pressure packaging modes, but is not limited thereto. The hard packaging clamp 200 can include a top cover 240 and a base 260. A protrusion 280 and at least one ditch 290 are arranged on the base 260. The protrusion 280 is connected to the ditch 290. The ditch 290 includes an injection opening 220 through which the drug is injected in. The average diameter of the cross section of the ditch can be about 50 µm to 1000 µm, for example, 100 µm to 950 µm, 150 µm to 900 µm, 200 µm to 850 µm, 250 µm to 800 µm, 300 µm to 750 µm, 350 µm to 700 µm, 500 µm to 750 µm, 500 µm to 800 µm, etc., but the disclosure is not limited thereto.

In the present embodiment, the top cover 240 includes a recessed part, the shape and the position of the recessed part correspond to those of the protrusion 280. The eye wearing device 10 can be put in the protrusion 280 of the base 260, in which the average curvature radius of the protrusion 280 is similar to that of the eye wearing device 10 so that the eye wearing device 10 is clamped in the protrusion 280 when the base 260 and the top cover 240 are buckled. The difference between the average curvature radius of the protrusion 280 and the average curvature radius of the eye wearing device 10 is only about 1 mm, 0.8 mm, 0.5 mm, 0.3 mm or 0.1 mm, etc., but the disclosure is not limited thereto.

Then, after the position of the eye wearing device 10 is fixed by the hard packaging clamp 200, drug filling can be performed by an injector 300 at the injection opening 220 of the hard packaging clamp 200, the drug can flow into the eye wearing device 10 on the protrusion 280 through the ditch 290 from the injection opening 220, and further enters the reservoir 12 through the microchannel 14 of the eye wearing device 10. In detail, as shown in FIG. 7D, a splay guide opening 230 can be further arranged in the position of the injection opening 220, so that the injector 300 is conveniently aligned with the injection opening 220. The injector 300 can include but is not limited to a needle head. For example, a dropper or a pipetman can be adopted for injecting the drug. Additionally, a sealing film 250 (or a rubber plug) can be arranged in front of or behind the guide opening 230 so as to maintain the vacuum or negative pressure in a package of the hard packaging clamp 200. When the injector 300 is inserted into the injection opening 220, the sealing film 250 can be fractured, and the drug filling is further performed in the above-mentioned mode.

Then, in FIG. 7A, FIG. 7B and FIG. 7C, only two ditches 290 are drawn, but the required ditches can be practically arranged according to requirements, for example, four, six, eight and so on, but the disclosure is not limited thereto. Additionally, different effective doses of drugs can be further injected through different injection openings, so that different kinds of required drugs are loaded in different reservoirs of the eye wearing device. For example, the eye wearing device 20 can be used as a drug loading object to be put in the protrusion 280 of the hard packaging clamp 200, one microchannel 24 respectively arranged in different reservoirs 22 faces one ditch 290, so that different drugs are delivered into the corresponding microchannel 24 by different ditches 290, and different drugs are loaded in different reservoirs 22 of the eye wearing device 20.

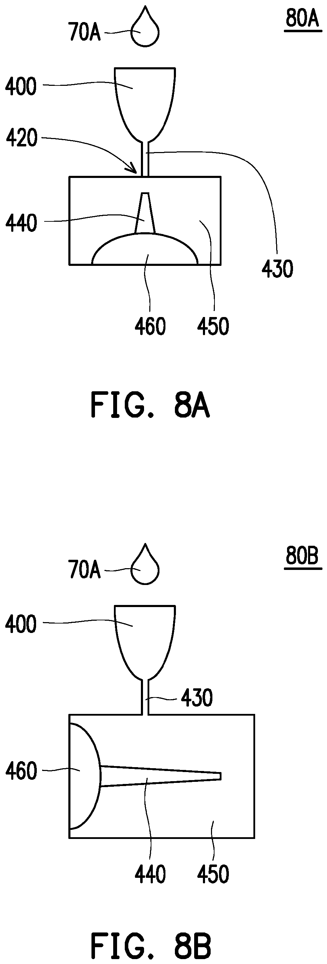

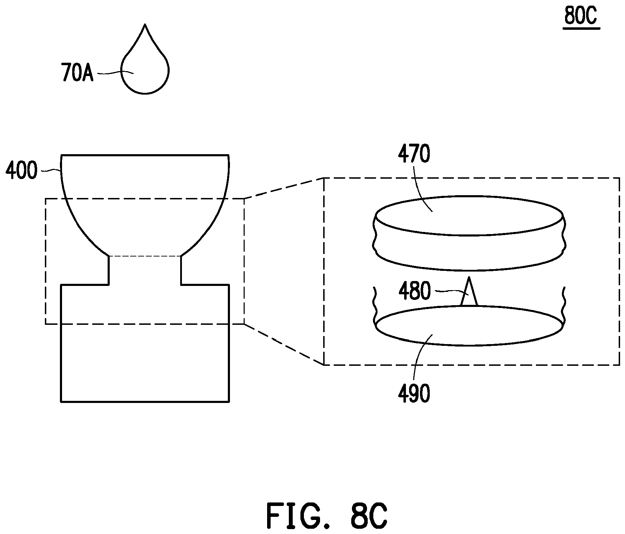

FIG. 8A, FIG. 8B and FIG. 8C are schematic diagrams of another package design and use method of the eye wearing device according to the disclosure.

In addition to the package design and use method of the eye wearing device illustrated in FIG. 7A, FIG. 7B and FIG. 7C, in order to avoid the filling by injection, the package designs shown in FIG. 8A, FIG. 8B and FIG. 8C can also be used. For the package designs in FIG. 8A, FIG. 8B and FIG. 8C, a drug drop container is used as a main device, which can replace the injector 300 in FIG. 7A, FIG. 7B and FIG. 7C and can be matched with the hard packaging clamp 200 in FIG. 7A, FIG. 7B and FIG. 7C to be used so as to fill effective doses of drugs into the eye wearing device 10. Referring to FIG. 8A, a drug drop container 80A can include a containing part 400, a sealing film 420, a safe invisible needle 440 and a storage space 450, in which the storage space 450 is arranged below the sealing film 420, and the safe invisible needle 440 can be arranged in the storage space 450. A connecting pipe 430 is arranged between the containing part 400 and the sealing film 420, one end of the connecting pipe 430 is connected to the bottom of the containing part 400, and the other end of the connecting pipe 430 is provided with the sealing film 420 configured to seal the opening. Additionally, a soft pad 460 can be arranged at the bottom of the safe invisible needle 440 in the storage space 450, for example, a plastic soft pad, so that the operation of the pressing action of the safe invisible needle 440 is convenient. The soft pad is favourable for the rebounding after the pressing action, so that the condition of hindering the drug from flowing to the storage space 450 from the connecting pipe 430 is avoided.

In operation, firstly, a drug 70A can be dropped into the containing part 400. After a user drops the drug 70A into the containing part 400 according to recommended doses, the soft pad 460 below the safe invisible needle 440 is pressed in a direction towards the sealing film 420 so that the safe invisible needle 440 pierces the sealing film 420, an effective dose of the drug 70A overflow into the storage space 450 through the connecting pipe 430 from the containing part 400. Then, referring to FIG. 8A and FIG. 7C at the same time, the drug 70A in the storage space 450 can be injected into the injection opening 220 of the hard packaging clamp 200 connected to the storage space 450 to enter the ditch 290 of the hard packaging clamp 200. The volume of the storage space 450 is smaller than that of the containing part 400, but is much greater than that of the reservoir 12 and the microchannel 14 in the eye wearing device 10, so that the problem of not filling up drug cannot occur.

Referring to FIG. 8B, a structure and an operation method of FIG. 8B are similar to those of FIG. 8A. The difference is that no sealing film 420 is arranged in a drug drop container 80B. In other words, the connecting pipe 430 is communicated with the storage space 450. The soft pad 460 can be arranged at a side surface. Meanwhile, the safe invisible needle 440 steers. In operation, the drug 70A is firstly dropped into the containing part 400 according to the recommended doses. Then, referring to FIG. 8B, FIG. 7C and FIG. 7D at the same time, the soft pad 460 is pressed in a direction towards the injection opening 220 of the hard packaging clamp 200, so that the safe invisible needle 440 can pierce the sealing film 250 of the injection opening 220, the effective dose of the drug 70A can overflow into the storage space 450 through the connecting pipe 430 from the containing part 400 through negative pressure, and is then injected into the injection opening 220 of the hard packaging clamp 200 connected to the storage space 450 through the storage space 450 and then enters the ditch 290 of the hard packaging clamp 200.

Referring to FIG. 8C, a structure and an operation method in FIG. 8C are similar to those in FIG. 8A. The difference is that in a drug drop container 80C, the connecting pipe 430, the sealing film 420, the safe invisible needle 440 and the soft pad 460 in FIG. 8A are replaced by a screw cap 470 with a sealing film and a support element 490 with a sharp object 480 (such as the safe invisible needle, a scraper or other sharp articles capable of being used for damaging the sealing film). In operation, the drug 70A is dropped into the containing part 400 firstly in the same way according to the recommended doses. Then, referring to FIG. 8C and FIG. 7C at the same time, the screw cap 470 is downwards screwed so that the sharp object 480 damages the sealing film in the screw cap 470, and the effective dose of the drug overflows into the storage space below the containing part 400 through a damage opening, and is then injected into the injection opening 220 of the hard packaging clamp 200 connected to the storage space through the storage space and then enters the ditch 290 of the hard packaging clamp 200.

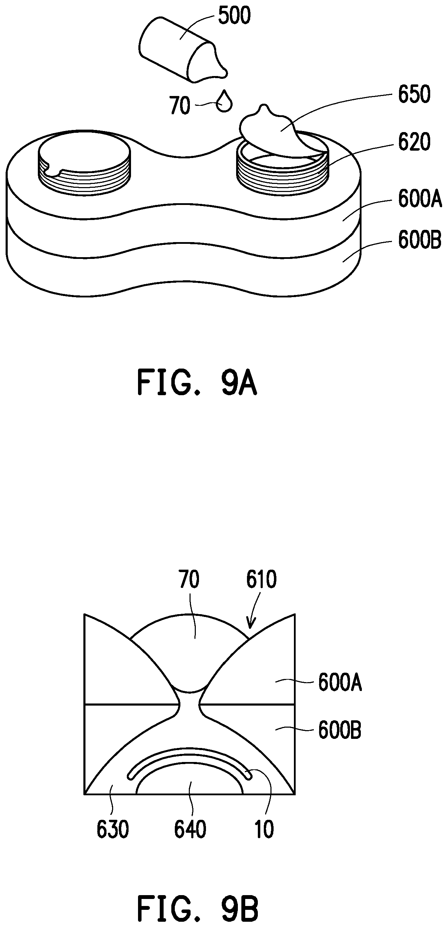

FIG. 9A and FIG. 9B are schematic diagrams of yet another package design and use method of the eye wearing device according to the disclosure, in which FIG. 9A is a stereoscopic schematic diagram, and FIG. 9B is a cross-section schematic diagram.

Structures in FIG. 9A and FIG. 9B are similar to those of hard packaging clamps 200 in FIG. 7A, FIG. 7B and FIG. 7C. The difference is that the structure is designed in a form like a contact lenses box, and is used by being preferably matched with the drug drop container 80C in FIG. 8C. Referring to FIG. 9A and FIG. 9B at the same time, an isolation film 650 configured to prevent pollution is torn away at first, then, the drug 70 is dropped into a drug drop container 620 according to the recommended doses by using a drop tool 500, and the drug drop container 620 here is similar to the drug drop container 80C in FIG. 8C. The drug drop container 620 is rotated so that a drug containing space 610 positioned in a top cover 600A is disconnected from a storage space 630 positioned in a base 600B, the drug 70 flows into the storage space 630 of the base 600B through the drug containing space 610 of the top cover 600A. A protrusion 640 is arranged in the storage space 630, the eye wearing device 10 can be put over the protrusion 640 of the storage space 630, and the average curvature radius of the protrusion 640 is similar to the average curvature radius of the eye wearing device 10. Therefore, the drug 70 in the storage space 630 can be filled into the eye wearing device 10.

In order to prove that the eye wearing device of the disclosure can effectively and slowly release a treatment drug, practical tests are respectively performed by aiming at the eye wearing devices of different modes designed by the disclosure hereafter, comparison to the existing drug-impregnated contact lenses is further performed, and operation and results of the experiment are shown hereafter in details.

The experiments are performed by aiming at the existing drug-impregnated contact lenses (Comparative example 1), the eye wearing device (Experimental example 1) in FIG. 1A, the eye wearing device similar to the eye wearing device in FIG. 1A but only provided with two channels (Experimental example 2, six openings of the eye wearing device in FIG. 1A are sealed by adhesive tapes), the eye wearing device in FIG. 6C (Experimental example 3) and the eye wearing device in FIG. 5A (Experimental example 4), and release constant and release time of 90% of the drugs of each comparative example and each experimental example are tested.

An experimental method is shown as follows: a microchannel prototype product is made of PDMS and hydrophilic treatment is performed. Then, pure water is used for cleaning the prototype product and ventilation and drying are performed. The PDMS prototype product sucking a drug solution is slowly put into 10 mL of pure water to prevent from perturbance. After that, the room temperature is maintained and sampling is performed every hour to every several hours, in which the longest sampling time reaches three days, and the volume of each sampling is 150 µL. During the sampling, the water is taken while stirring is slowly performed, and a hole passage opening is avoided as well as the time is recorded. Later, the light absorbance value of the drug is read by a UV/VIS spectrophotometer, and a standard drug concentration curve is made for concentration comparison. The test results are shown as Table 1.

As can be seen from Table 1, compared with the existing drug-impregnated contact lenses (Comparative example 1), the eye wearing devices (Experimental example 1, Experimental example 2, Experimental example 3 and Experimental example 4) designed according to the disclosure can effectively and slowly release the treatment drug.

**Table 1**

| | Comparative example 1 | Experimental example 1 | Experimental example 2 | Experimental example 3 | Experimental example 4 |
|---|---|---|---|---|---|
| Release constant | 38.4 | 14 | 3 | 3.6 | 1.9 |
| Release time of 90% of drugs (hr) | 1 | 2.5 | 12 | 7.5 | 20 |

Although the disclosure is described with reference to the above embodiments, the embodiments are not intended to limit the disclosure. The protection scope of the disclosure should be subject to the appended claims.

## Claims

1. An eye wearing device, comprising:
a body (P), having a first surface (S1), a second surface (S2), a center (N10), a first outer edge (E1) of the first surface (S1) and a second outer edge (E2) of the second surface (S2);
at least one reservoir (12), arranged in the body (P) and configured to load a drug; the eye wearing device being **characterised in** further comprising
at least one microchannel (14), arranged close to the first outer edge (E1) of the first surface (S1) and the second outer edge (E2) of the second surface (S2), wherein one end of the microchannel (14) is connected to the reservoir (12) so as to fill the drug into the reservoir (12), and the other end of the microchannel (14) is an opening (O) facing an edge of the eye wearing device (10), and configured to be in contact with a cornea and/or a sclera, and the opening (O) is connected to the first outer edge (E1) of the first surface (S1) and the second outer edge (E2) of the second surface (S2),
wherein an average diameter of a cross section of the microchannel (14) is smaller than or equal to an average diameter of a cross section of the reservoir (12), an average curvature radius of the eye wearing device (10) is 6 mm to 15 mm, and the eye wearing device (10) is worn on the cornea and/or the sclera of a user through the second surface (S2).

2. The eye wearing device according to claim 1, wherein the reservoir (12) has the width of 30 µm to 5 mm, the height of 10 µm to 200 µm, the total volume of 0.002 µL to 20 µL, and an average diameter of a cross section of the microchannel (14) is 20 µm to 150 µm.

3. The eye wearing device according to claim 1, wherein a diameter of the eye wearing device (10) is 12 mm to 20 mm, and an average thickness of the eye wearing device (10) is 20 µm to 400 µm.

4. The eye wearing device according to claim 1, wherein a diameter of the microchannel (14) is gradually reduced from the edge of the eye wearing device (10) to the reservoir (12).

5. The eye wearing device according to claim 1, wherein a diameter of one end of the microchannel (14) connected to the reservoir (12) is 10 µm to 200 µm, and a diameter of another end of the microchannel (14) in contact with the cornea and/or the sclera is 40 µm to 200 µm.

6. The eye wearing device according to claim 1, wherein a lubricating material is coated on a surface of the eye wearing device (10), the lubricating material comprises mucoprotein, polyethylenimine, polyethylene glycol, polyacrylic acid, polymethacrylic acid, polyitaconic acid, polymaleic acid, carboxymethyl cellulose (CMC), hydroxypropyl methylcellulose (HPMC), polyvinylpyrrolidone, polyacrylamide, poleyvinylalcohol, hyaluronic acid, dextran, poly 2-hydroxyethyl methacrylate (poly HEMA), poly sulfonates, polylactate, urea, phosphoryl choline or a combination thereof.

7. The eye wearing device according to claim 6, wherein the lubricating material further comprises hydrophilic polypeptides, and amino acid in an amount of 75% or more by weight of the hydrophilic polypeptides is selected from a group consisting of aspartic acid (Asp or D), glutamic acid (Glu or E), histidine (His or H), lysine (Lys or K), asparagine (Asn or N), glutamine (Gln or Q), arginine (Arg or R), serine (Ser or S), threonine (Thr or T) and tyrosine (Tyr or Y).

8. The eye wearing device according to claim 1, wherein the material of the eye wearing device (10) comprises bio-derived polymers, non-bio-derived polymers or a combination thereof.

9. The eye wearing device according to claim 8, wherein the bio-derived polymers comprise collagen, gelatin, chitin, cellulose or a combination thereof.

10. The eye wearing device according to claim 8, wherein the non-bio-derived polymers comprise polyethylene glycol (PEG), propylene glycol diacrylate (PPGDA), polydimethylsiloxane (PDMS), poly(methyl methacrylate) (PMMA), poly(hydroxyethyl methacrylate) (PHEMA) or a combination thereof.

11. The eye wearing device according to claim 1, further comprising a package for loading the eye wearing device (10), the package comprises a hard packaging clamp (200), and the hard packaging clamp (200) comprises:
a base (260), wherein a protrusion (280) and at least one ditch (290) are arranged on the base (260), the protrusion (280) is connected to the ditch (290), and the protrusion (280) is configured to place the eye wearing device (10), and the ditch (290) has an injection opening (220) for injection of the drug; and
a top cover (240), having a recessed part, wherein the shape and the position of the recessed part correspond to those of the protrusion (280),
wherein an average curvature radius of the protrusion (280) is similar to an average curvature radius of the eye wearing device (10), so that the eye wearing device (10) is clamped in the protrusion (280) when the base (260) and the top cover (240) are buckled, and an average diameter of a cross section of the ditch (290) is 50 µm to 1000 µm.

12. The eye wearing device according to claim 11, wherein the package further comprises:
at least one drug drop container (80A, 80B, 80C) configured to load an effective dose of the drug.

13. The eye wearing device according to claim 12, wherein the drug drop container (80A, 80B) comprises:
a containing part (400);
a connecting pipe (430), connected to the bottom of the containing part (400);
a storage space (450), arranged below the connecting pipe (430) and connected to the injection opening (220) of the hard packaging clamp (200); and
a safe invisible needle (440), arranged in the storage space (450).

14. The eye wearing device according to claim 13, wherein a sealing film (420) is arranged between the storage space (450) and the connecting pipe (430), and the drug overflows into the storage space (450) through the connecting pipe (430) from the containing part (400) when the safe invisible needle (440) is pressed, and is then injected into the injection opening (220) from the storage space (450) to enter the ditch (290) of the hard packaging clamp (200).

15. The eye wearing device according to claim 12, wherein the drug drop container (80C) comprises:
a screw cap (470), configured with a sealing film;
a support element (490), configured with a sharp object (480); and
a storage space, arranged below the support element (490) and connected to the injection opening (220) of the hard packaging clamp (200),
wherein after the screw cap (470) is downwards screwed, the sharp object (480) damages the sealing film in the screw cap (470), so that the drug overflows into the storage space and is then injected into the injection opening (220) from the storage space to enter the ditch (290) of the hard packaging clamp (200).

## Patentansprüche

1. Augentragevorrichtung, umfassend:
einen Körper (P), der eine erste Oberfläche (S1), eine zweite Oberfläche (S2), eine Mitte (N10), eine erste Außenkante (E1) der ersten Oberfläche (S1) und eine zweite Außenkante (E2) der zweiten Oberfläche (S2) aufweist;
mindestens ein Reservoir (12), das in dem Körper (P) angeordnet und zum Laden eines Medikaments konfiguriert ist, wobei die Augentragevorrichtung **dadurch gekennzeichnet ist, dass** sie ferner umfasst:
mindestens einen Mikrokanal (14), der nahe der ersten Außenkante (E1) der ersten Oberfläche (S1) und der zweiten Außenkante (E2) der zweiten Oberfläche (S2) angeordnet ist, wobei ein Ende des Mikrokanals (14) mit dem Reservoir (12) verbunden ist, um das Medikament in das Reservoir (12) zu füllen und das andere Ende des Mikrokanals (14) eine Öffnung (O) ist, die einer Kante der Augentragevorrichtung (10) zugewandt ist, und so konfiguriert, um in Kontakt mit einer Hornhaut und/oder einer Sklera zu sein, und die Öffnung (O) mit der ersten Außenkante (E1) der ersten Oberfläche (S1) und der zweiten Außenkante (E2) der zweiten Oberfläche (S2) verbunden ist,
wobei ein durchschnittlicher Durchmesser eines Querschnitts des Mikrokanals (14) kleiner oder gleich einem durchschnittlichen Durchmesser eines Querschnitts des Reservoirs (12) ist, ein durchschnittlicher Krümmungsradius der Augentragevorrichtung (10) 6 mm bis 15 mm beträgt und die Augentragevorrichtung (10) durch die zweite Oberfläche (S2) auf der Hornhaut und/oder der Sklera eines Benutzers getragen wird.

2. Augentragevorrichtung gemäß Anspruch 1, wobei das Reservoir (12) eine Breite von 30 µm bis 5 mm, eine Höhe von 10 µm bis 200 µm, ein Gesamtvolumen von 0,002 µL bis 20 µL aufweist und ein durchschnittlicher Durchmesser eines Querschnitts des Mikrokanals (14) von 20 µm bis 150 µm beträgt.

3. Augentragevorrichtung gemäß Anspruch 1, wobei ein Durchmesser der Augentragevorrichtung (10) 12 mm bis 20 mm beträgt, und eine durchschnittliche Dicke der Augentragevorrichtung (10) 20 µm bis 400 µm beträgt.

4. Augentragevorrichtung gemäß Anspruch 1, wobei sich ein Durchmesser des Mikrokanals (14) von der Kante der Augentragevorrichtung (10) zum Reservoir (12) hin allmählich verringert.

5. Augentragevorrichtung gemäß Anspruch 1, wobei ein Durchmesser eines Endes des Mikrokanals (14), das mit dem Reservoir (12) verbunden ist, 10 µm bis 200 µm beträgt, und ein Durchmesser eines anderen Endes des Mikrokanals (14), das in Kontakt mit der Hornhaut und/oder der Sklera steht, 40 µm bis 200 µm beträgt.

6. Augentragevorrichtung gemäß Anspruch 1, wobei ein Gleitmaterial auf eine Oberfläche der Augentragevorrichtung (10) aufgetragen ist, wobei das Gleitmaterial Mucoprotein, Polyethylenimin, Polyethylenglykol, Polyacrylsäure, Polymethacrylsäure, Polyitaconsäure, Polymaleinsäure, Carboxymethylcellulose (CMC), Hydroxypropylmethylcellulose (HPMC), Polyvinylpyrrolidon, Polyacrylamid, Polyvinylalkohol, Hyaluronsäure, Dextran, Poly-2-hydroxyethylmethacrylat (Poly HEMA), Polysulfonate, Polylactat, Harnstoff, Phosphorylcholin oder eine Kombination davon umfasst.

7. Augentragevorrichtung gemäß Anspruch 6, wobei das Gleitmaterial weiterhin hydrophile Polypeptide umfasst und Aminosäure, die in einer Menge von 75 Gew.-% oder mehr der hydrophilen Polypeptide, ausgewählt ist aus einer Gruppe bestehend aus Asparaginsäure (Asp oder D), Glutaminsäure (Glu oder E), Histidin (His oder H), Lysin (Lys oder K), Asparagin (Asn oder N), Glutamin (Gln oder Q), Arginin (Arg oder R), Serin (Ser oder S), Threonin (Thr oder T) und Tyrosin (Tyr oder Y).

8. Augentragevorrichtung gemäß Anspruch 1, wobei das Material der Augentragevorrichtung (10) biologisch abgeleitete Polymere, nicht-biologisch abgeleitete Polymere oder eine Kombination davon umfasst.

9. Augentragevorrichtung gemäß Anspruch 8, wobei die biologisch abgeleiteten Polymere Kollagen, Gelatine, Chitin, Cellulose oder eine Kombination davon umfassen.

10. Augentragevorrichtung gemäß Anspruch 8, wobei die nicht-biologisch abgeleiteten Polymere Polyethylenglykol (PEG), Propylenglykoldiacrylat (PPGDA), Polydimethylsiloxan (PDMS), Poly(methylmethacrylat) (PMMA), Poly(hydroxyethylmethacrylat) (PHEMA) oder eine Kombination davon umfassen.

11. Augentragevorrichtung gemäß Anspruch 1, ferner umfassend eine Verpackung zum Laden der Augentragevorrichtung (10), wobei die Verpackung eine Hartverpackungsklammer (200) umfasst, und die Hartverpackungsklammer (200) umfasst:
eine Basis (260), wobei ein Vorsprung (280) und mindestens ein Graben (290) an der Basis (260) angeordnet sind, der Vorsprung (280) mit dem Graben (290) verbunden ist, und der Vorsprung (280) konfiguriert ist, um die Augentragevorrichtung (10) zu platzieren, und der Graben (290) eine Injektionsöffnung (220) für die Injektion des Medikaments aufweist; und
eine obere Abdeckung (240), die einen ausgesparten Teil aufweist, wobei die Form und die Position des ausgesparten Teils der Form und der Position des Vorsprungs (280) entsprechen,
wobei ein durchschnittlicher Krümmungsradius des Vorsprungs (280) ähnlich einem durchschnittlichen Krümmungsradius der Augentragevorrichtung (10) ist, so dass die Augentragevorrichtung (10) in dem Vorsprung (280) zusammengeschnallt ist, wenn die Basis (260) und die obere Abdeckung (240) geknickt sind, und ein durchschnittlicher Durchmesser eines Querschnitts des Grabens (290) 50 µm bis 1000 µm beträgt.

12. Augentragevorrichtung gemäß Anspruch 11, wobei die Verpackung weiterhin umfasst:
mindestens einen Arzneimitteltropfbehälter (80A, 80B, 80C), der so konfiguriert ist, dass er eine wirksame Dosis des Medikaments lädt.

13. Augentragevorrichtung gemäß Anspruch 12, wobei der Medikamententropfbehälter (80A, 80B) umfasst:
ein Aufnahmeteil (400);
ein Verbindungsrohr (430), das mit dem Boden des Aufnahmeteils (400) verbunden ist;
einen Speicherraum (450), der unterhalb des Verbindungsrohrs (430) angeordnet und mit der Injektionsöffnung (220) der Hartverpackungsklammer (200) verbunden ist; und
eine sichere, unsichtbare Nadel (440), die in dem Speicherraum (450) angeordnet ist.

14. Augentragevorrichtung gemäß Anspruch 13, wobei zwischen dem Speicherraum (450) und dem Verbindungsrohr (430) eine Dichtungsfolie (420) angeordnet ist und das Medikament, wenn die sichere, unsichtbare Nadel (440) gedrückt wird, durch das Verbindungsrohr (430) aus dem Aufnahmeteil (400) in den Speicherraum (450) überläuft und dann aus dem Speicherraum (450) in die Injektionsöffnung (220) injiziert wird, um in den Graben (290) der Hartverpackungsklammer (200) einzutreten.

15. Augentragevorrichtung gemäß Anspruch 12, wobei der Medikamententropfbehälter (80C) umfasst:
eine Schraubkappe (470), die mit einer Dichtungsfolie ausgestattet ist;
ein Stützelement (490), das mit einem scharfen Gegenstand (480) konfiguriert ist; und
einen Speicherraum, der unterhalb des Stützelements (490) angeordnet und mit der Injektionsöffnung (220) der Hartverpackungsklammer (200) verbunden ist,
wobei nach dem Herunterschrauben der Schraubkappe (470) der scharfe Gegenstand (480) die Dichtungsfolie in der Schraubkappe (470) beschädigt, so dass das Medikament in den Speicherraum überläuft und dann aus dem Speicherraum in die Injektionsöffnung (220) injiziert wird, um in den Graben (290) der Hartverpackungsklammer (200) einzutreten.

## Revendications

1. Dispositif à porter sur l'œil, comprenant :
un corps (P), ayant une première surface (S1), une seconde surface (S2), un centre (N10), un premier bord extérieur (E1) de la première surface (S1) et un second bord extérieur (E2) de la seconde surface(S2) ;
au moins un réservoir (12), agencé dans le corps (P) et configuré pour charger un médicament ; le dispositif à porter sur l'œil étant **caractérisé en ce qu'**il comprend en outre
au moins un microcanal (14), agencé à proximité du premier bord extérieur (E1) de la première surface (S1) et du second bord extérieur (E2) de la seconde surface (S2), dans lequel une extrémité du microcanal (14) est raccordée au réservoir (12) de manière à remplir le médicament dans le réservoir (12), et l'autre extrémité du microcanal (14) est une ouverture (O) face à un bord du dispositif à porter sur l'œil (10), et configuré pour être en contact avec une cornée et/ou une sclère, et l'ouverture (O) est raccordée au premier bord extérieur (E1) de la première surface (S1) et au second bord extérieur (E2) de la seconde surface (S2),
dans lequel un diamètre moyen d'une section transversale du microcanal (14) est inférieur ou égal à un diamètre moyen d'une section transversale du réservoir (12), un rayon de courbure moyen du dispositif à porter sur l'œil (10) est de 6 mm à 15 mm, et le dispositif à porter sur l'œil (10) est porté sur la cornée et/ou la sclère d'un utilisateur par l'intermédiaire de la seconde surface (S2).

2. Dispositif à porter sur l'œil selon la revendication 1, dans lequel le réservoir (12) a la largeur de 30 µm à 5 mm, la hauteur de 10 µm à 200 µm, le volume total de 0,002 µl à 20 pl, et un diamètre moyen d'une section transversale du microcanal (14) est de 20 µm à 150 µm.

3. Dispositif à porter sur l'œil selon la revendication 1, dans lequel un diamètre du dispositif à porter sur l'œil (10) est de 12 mm à 20 mm, et une épaisseur moyenne du dispositif à porter sur l'œil (10) est de 20 µm à 400 µm.

4. Dispositif à porter sur l'œil selon la revendication 1, dans lequel un diamètre du microcanal (14) est progressivement réduit du bord du dispositif à porter sur l'œil (10) au réservoir (12).

5. Dispositif à porter sur l'œil selon la revendication 1, dans lequel un diamètre d'une extrémité du microcanal (14) raccordé au réservoir (12) est de 10 µm à 200 µm, et un diamètre d'une autre extrémité du microcanal (14) en contact avec la cornée et/ou la sclère est de 40 µm à 200 µm.

6. Dispositif à porter sur l'œil selon la revendication 1, dans lequel un matériau lubrifiant est revêtu sur une surface du dispositif à porter sur l'œil (10), le matériau lubrifiant comprend une mucoprotéine, une polyéthylènimine, un polyéthylène glycol, un acide polyacrylique, un acide polyméthacrylique, un acide polyitaconique, un acide polymaléique, une carboxyméthyl cellulose (CMC), une hydroxypropyl méthylcellulose (HPMC), une polyvinylpyrrolidone, un polyacrylamide, un alcool polyvinylique, un acide hyaluronique, un dextrane, un poly-méthacrylate de 2-hydroxyéthyle (poly HEMA), des poly sulfonates, un polylactate, une urée, une phosphoryl choline ou une combinaison de ceux-ci.

7. Dispositif à porter sur l'œil selon la revendication 6, dans lequel un matériau lubrifiant comprend en outre des peptides hydrophiles, et un acide aminé en une quantité de 75 % ou plus en poids des polypeptides hydrophiles est sélectionné dans un groupe consistant en l'acide aspartique (Asp ou D), l'acide glutamique (Glu ou E), l'histidine (His ou H), la lysine (Lys ou K), l'asparagine (Asn ou N), la glutamine (Gln ou Q), l'arginine (Arg ou R), la sérine (Ser ou S), la thréonine (Thr ou T) et la tyrosine (Tyr ou Y).

8. Dispositif à porter sur l'œil selon la revendication 1, dans lequel le matériau du dispositif à porter sur l'œil (10) comprend des polymères bio-dérivés, des polymères non bio-dérivés ou une combinaison de ceux-ci.

9. Dispositif à porter sur l'œil selon la revendication 8, dans lequel les polymères bio-dérivés comprennent le collagène, la gélatine, la chitine, la cellulose ou une combinaison de ceux-ci.

10. Dispositif à porter sur l'œil selon la revendication 8, dans lequel les polymères non-biodérivés comprennent le polyéthylène glycol (PEG), le diacrylate de propylène glycol (PPGDA), le polydiméthylsiloxane (PDMS), le poly(méthacrylate de méthyle) (PMMA), le poly(méthacrylate d'hydroxyéthyle) (PHEMA) ou une combinaison de ceux-ci.

11. Dispositif à porter sur l'œil selon la revendication 1, comprenant en outre un emballage pour charger le dispositif à porter sur l'œil (10), l'emballage comprend une pince d'emballage dure (200), et la pince d'emballage dure (200) comprend :
une base (260), dans une saillie (280) et au moins une rainure (290) sont agencées sur la base (260), la saillie (280) est raccordée à la rainure (290), et la saillie (280) est configurée pour placer le dispositif à porter sur l'œil (10), et la rainure (290) a une ouverture d'injection (220) pour l'injection du médicament ; et
une couverture supérieure (240), ayant une partie encastrée, dans lequel la forme et la position de la partie encastrée correspondent à celles de la saillie (280),
dans lequel un rayon de courbure de la saillie (280) est similaire à un rayon de courbure moyen du dispositif à porter sur l'œil (10), de manière à ce que le dispositif à porter sur l'œil (10) soit serré dans la saillie (280) lorsque la base (260) et la couverture supérieure (240) sont pliées, et un diamètre moyen d'une section transversale de la rainure (290) et de 50 µm à 1000 µm.

12. Dispositif à porter sur l'œil selon la revendication 11, dans lequel l'emballage comprend en outre :
au moins un récipient pour gouttes oculaires (80A, 80B, 80C) configuré pour charger une dose efficace du médicament.

13. Dispositif à porter sur l'œil selon la revendication 12, dans lequel le récipient pour gouttes oculaires (80A, 80B) comprend :
une partie de réception (400) ;
un tube de raccordement (430), raccordé au fond de la partie de réception (400) ;
un espace de stockage (450), agencé au-dessous du tube de raccordement (430) et raccordé à l'ouverture d'injection (220) de la pince d'emballage dure (200) ; et
une aiguille invisible sûre (440), agencée dans l'espace de stockage (450).

14. Dispositif à porter sur l'œil selon la revendication 13, dans lequel un film d'étanchéité (420) est agencé entre l'espace de stockage (450) et le tube de raccordement (430), et le médicament se déverse dans l'espace de stockage (450) par l'intermédiaire du tube de raccordement (430) depuis la partie de réception (400) lorsque l'aiguille invisible sûre (440) est pressée, puis est alors injecté dans l'ouverture d'injection (220) depuis l'espace de stockage (450) pour entrer dans la rainure (290) de la pince d'emballage sûre (200).

15. Dispositif à porter sur l'œil selon la revendication 12, dans lequel le récipient pour gouttes oculaires (80C) comprend :
un capuchon à vis (470), configuré avec un film d'étanchéité ;
un élément de support (490), configuré avec un objet pointu (480) ; et
un espace de stockage, agencé au-dessous de l'élément de support (490) et raccordé à l'ouverture d'injection (220) de la pince d'emballage dure (200),
dans lequel une fois que le capuchon à vis (470) est vissé vers le bas, l'objet pointu (480) endommage le film d'étanchéité dans le capuchon à vis (470), de telle sorte que le médicament se déverse dans l'espace de stockage puis est alors injecté dans l'ouverture d'injection (220) depuis l'espace de stockage pour entrer dans la rainure (290) de la pince d'emballage dure (200).
